# EUROPEAN PATENT APPLICATION

(11) **EP 2 700 917 A1**
(43) Date of publication of application: **26.02.2014**
(21) Application number: 13159703.1
(22) Date of filing: 18.03.2013
(51) Int. Cl.: G01G 19/44, G01G 21/28, G01G 19/50, A61B 5/00

(54) **Biological information measuring device**

(30) Priority: 21.08.2012 JP 2012182243
(71) Applicant: Tanita Corporation, Tokyo 174-8630 (JP)
(72) Inventor: Hatakeyama, Minoru, Itabashi-ku, Tokyo 174-8630 (JP)
(74) Representative: Haley, Stephen

(57) **Abstract**

In a body composition monitor (10), a display unit holder (30) is provided at the upper edge of a support column (20). The display unit (60) is connected to the display unit holder (30) so as to slide back and forth. Furthermore, the display unit (60) can be connected to the display unit holder (30) while facing the opposite direction. The display unit (60) can slide back and forth when facing the opposite direction as well.

## Description

### CROSS REFERENCE TO RELATED APPLICATION

This application is based on an application No. 2012-182243 filed in Japan on August 21, 2012, the contents of which are hereby incorporated by reference.

### TECHNICAL FIELD

The present invention relates to biological information measuring devices, such as body composition monitors and scales, provided with a display.

### BACKGROUND ART

Body composition monitors that allow a user to operate a display unit while standing on a measurement unit, i.e. the main unit of the body composition monitor, are well known (for example, see Patent Literature 1). Specifically, a perpendicular support column is fixed at the front of the main unit of the body composition monitor. The display unit includes a touchscreen panel that displays a variety of information, such as the user's weight, and receives instructions from the user.

### CITATION LIST

Patent Literature

PTL 1: JP201011906A

### SUMMARY OF INVENTION

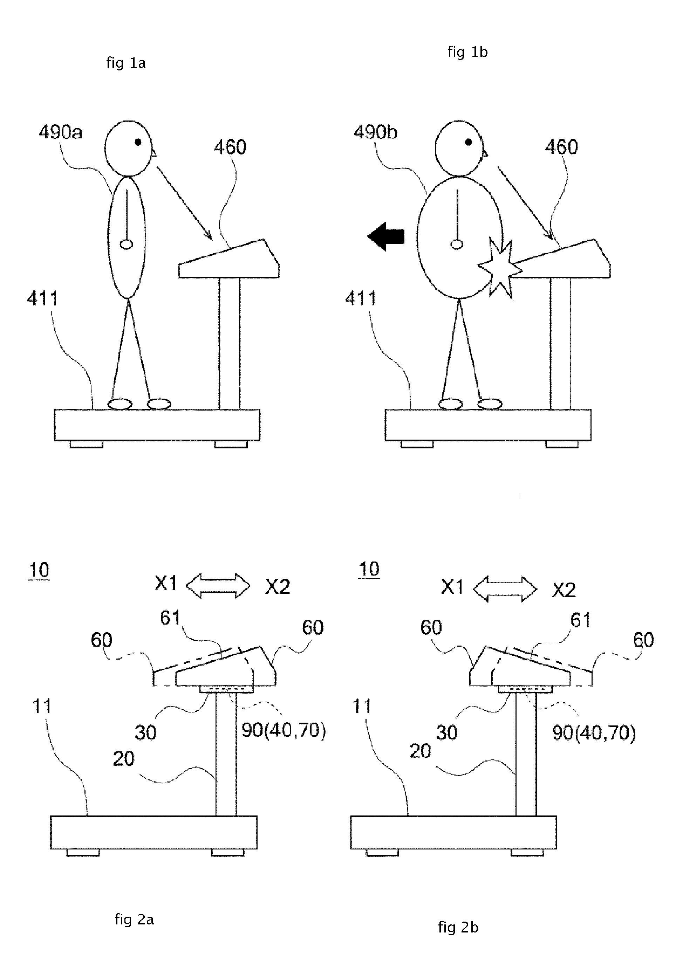

In a biological information measuring device such as a body composition monitor or scale, the display unit is fixed during use. Therefore, as shown in Fig. 1A, since a user 490a of average build can stand at an ideal position on the main unit of the body composition monitor (measurement stand) 411, the display unit 460 is at an appropriate distance for the user 490a. On the other hand, as shown in FIG. 1B, a well-built user 490b may bump into the display unit 460 and may therefore need to stand at the back of the main unit of the body composition monitor (measurement stand) 411. This leads to the problem of a greater distance between the user 490b and the display unit 460, thereby decreasing visibility and making the display unit 460 difficult to operate.

The present invention has been conceived in light of these circumstances, and it is an object thereof to provide a technique for solving the above problems.

A biological information measuring device according to the present invention comprises a platform on which a user stands for measurement; a display unit disposed at an upper side of the platform; and a sliding connector connecting the display unit to the upper side of the platform so that the display unit slides back and forth to predetermined positions.
The sliding connector may allow for reversal of a state of connection of the display unit, the display unit sliding back and forth in the reversed state.
The sliding connector may include a guide portion that guides sliding of the display unit back and forth.
A support column provided on the platform, and a display unit holder provided on the support column and receiving the display unit may be further provided. The sliding connector may include a first connecting element provided on the display unit and a second connecting element provided on the display unit holder, the guide portion may be provided on the display unit holder, and the first connecting element and the second connecting element may latch by a latch claw, provided on one of the first connecting element and the second connecting element, fitting into an opening provided on the other of the first connecting element and the second connecting element and configured to engage with the latch claw.
A display unit holder holding the display unit at the upper side of the platform may be further provided. The sliding connector may include a front latch hole placement convexity and a back latch hole placement convexity which are provided on the display unit, as well as a central latch portion, a front side-latch portion, and a back side-latch portion which are provided on the display unit holder. When the display unit is held in the display unit holder facing forward and the display unit is slid towards a front of the platform, a back latch portion of the central latch portion may fit into a front latch hole portion of the back latch hole placement convexity, and the front side-latch portion may fit into a front latch hole portion of the front latch hole placement convexity. When the display unit is held in the display unit holder facing forward and the display unit is slid towards a back of the platform, a front latch portion of the central latch portion may fit into a back latch hole portion of the front latch hole placement convexity, and the back side-latch portion may fit into a back latch hole portion of the back latch hole placement convexity.
When the display unit is held in the display unit holder facing backward and the display unit is slid towards the front of the platform, the back latch portion of the central latch portion may fit into the back latch hole portion of the front latch hole placement convexity, and the front side-latch portion may fit into the back latch hole portion of the back latch hole placement convexity. When the display unit is held in the display unit holder facing backward and the display unit is slid towards the back of the platform, the front latch portion of the central latch portion may fit into the front latch hole portion of the back latch hole placement convexity, and the back side-latch portion may fit into the front latch hole portion of the front latch hole placement convexity.

According to the present invention, there is realized a technique for appropriately sliding the display unit of a biological information measuring device back and forth.

### BRIEF DESCRIPTION OF DRAWINGS

The present invention will be further described below with reference to the accompanying drawings, wherein:
FIG. 1A shows examples of forms of use of a conventional body composition monitor by a user of average build;
FIG. 1B shows examples of forms of use of a conventional body composition monitor by a well-built user;
FIG. 2A schematically shows side views of a body composition monitor with a display unit facing forward according to an embodiment;
FIG. 2B schematically shows side views of a body composition monitor with a display unit facing backward according to an embodiment;
FIG. 3 is a perspective view of the body composition monitor according to the embodiment;
FIG. 4 is a perspective view of a display unit holder according to the embodiment;
FIG. 5 is a perspective view from below of a display unit according to the embodiment;
FIG. 6A is a plan view of the state of connection of a display unit holder and a display unit according to the embodiment in a state of connection;
FIG. 6B is a B-B cross-sectional diagram of FIG. 6A;
FIG. 7 is a cross-sectional enlarged view of region A in FIG. 6(b) according to the embodiment;
FIG. 8A schematically shows first step for connecting the display unit according to the embodiment to face forward;
FIG. 8B schematically shows a second step for connecting the display unit according to the embodiment to face forward;
FIG. 8C schematically shows a third step for connecting the display unit according to the embodiment to face forward;
FIG. 8D schematically shows a fourth step for connecting the display unit according to the embodiment to face forward;
FIG. 9A schematically shows a first step for connecting the display unit according to the embodiment to face backward;
FIG. 9B schematically shows a second step for connecting the display unit according to the embodiment to face backward;
FIG. 9C schematically shows a third step for connecting the display unit according to the embodiment to face backward;
FIG. 9D schematically shows a fourth step for connecting the display unit according to the embodiment to face backward;
FIG. 10 is a perspective view of a body composition monitor according to a modification of the embodiment;
FIG. 11 is a perspective view of the body composition monitor according to the modification of the embodiment;
FIG. 12A shows the bottom surface of display unit according to the modification of the embodiment;
FIG. 12B is a back perspective view from below of display unit in FIG. 12A; and
FIG. 12C is an enlarged view of region C in FIG. 12B.

### DESCRIPTION OF EMBODIMENTS

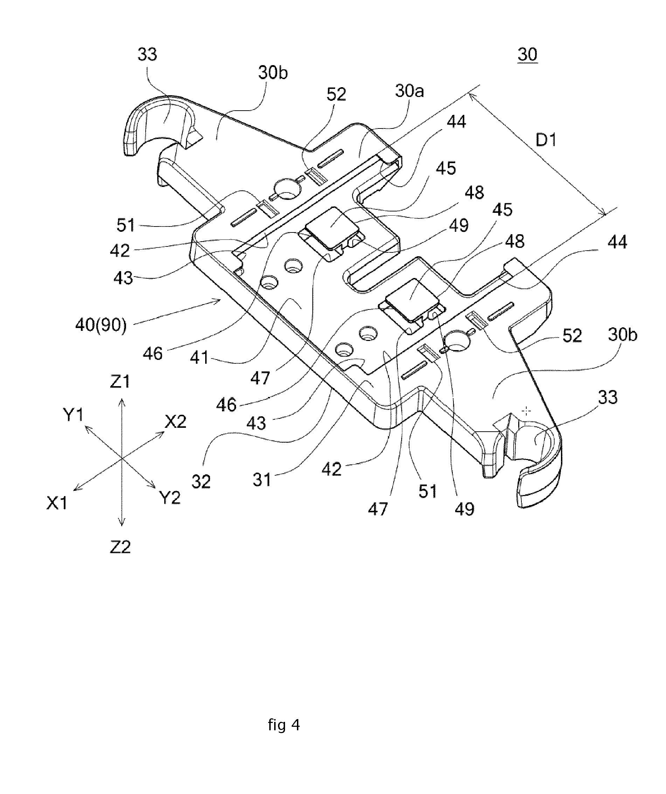

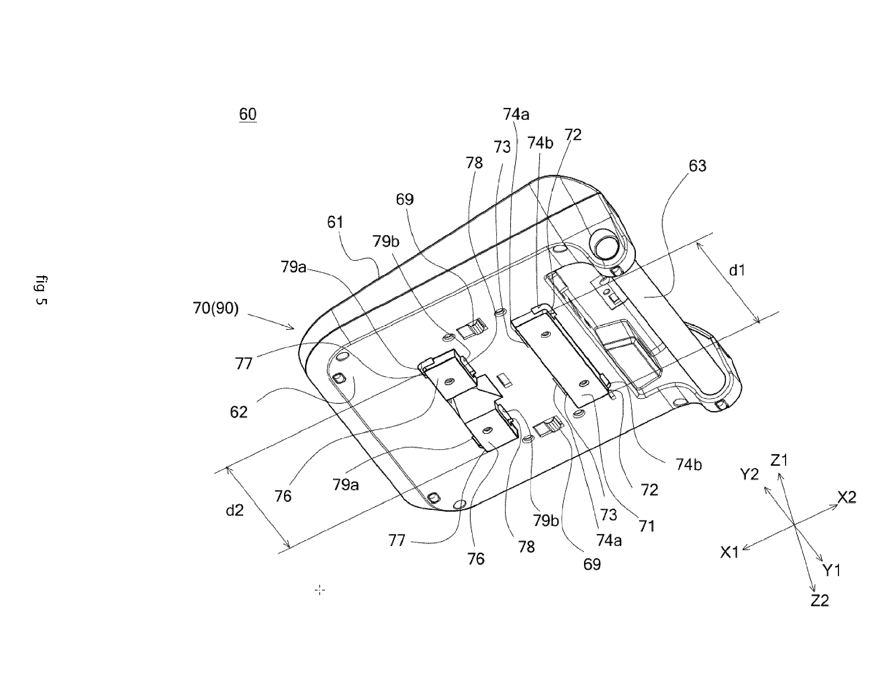

With reference to the drawings, the following describes an embodiment of the present invention in detail. First, an outline of the present embodiment is provided with reference to FIGS. 2A and 2B. In the present embodiment, a body composition monitor 10 is provided as an example of a biological information measuring device. However, devices in which the present embodiment may be adopted include scales, stabilometers, and the like. First, in the body composition monitor 10 of the present embodiment, a display unit holder 30 is provided at the upper edge of a support column 20, and a display unit 60 is connected to the display unit holder 30 so as to be able to slide back and forth, as shown by the schematic side views in FIG. 2. Furthermore, as shown in FIG. 2B, the display unit 60 can be removed and turned to face the opposite direction. Even when facing the opposite direction, the display unit 60 can slide back and forth once connected. The body composition monitor 10 includes a sliding connector 90 as the structure for connecting the display unit holder 30 and the display unit 60. Specifically, the sliding connector 90 includes a display unit connector 70 (first connecting element) provided on the display unit 60, described below with reference to FIG. 5, and a holder connector 40 (second connecting element) provided on the display unit holder 30, described below with reference to FIG. 4.

Note that the display unit 60 is considered to be facing forward in FIG. 2A and to be facing backward in FIG. 2B. Furthermore, in the figures, the arrow X1 is considered to point to the back (towards the user), the arrow X2 to point to the front (away from the user), the arrow Y1 to point to the left, and the arrow Y2 to point to the right. The arrow Z1 is considered to point vertically upward, and the arrow Z2 to point vertically downward.

When the display unit 60 faces forward as in FIG. 2A, a display unit upper surface 61 is inclined, with the back lower than the front. A display panel is provided on this inclined portion and thus is highly visible for a user standing on a measurement stand 11. On the other hand, when the display unit 60 faces backward as in FIG. 2B, the display panel is difficult for a user standing on the measurement stand 11 to see, yet is highly visible for another person, such as an operator, standing in front of the display unit 60.

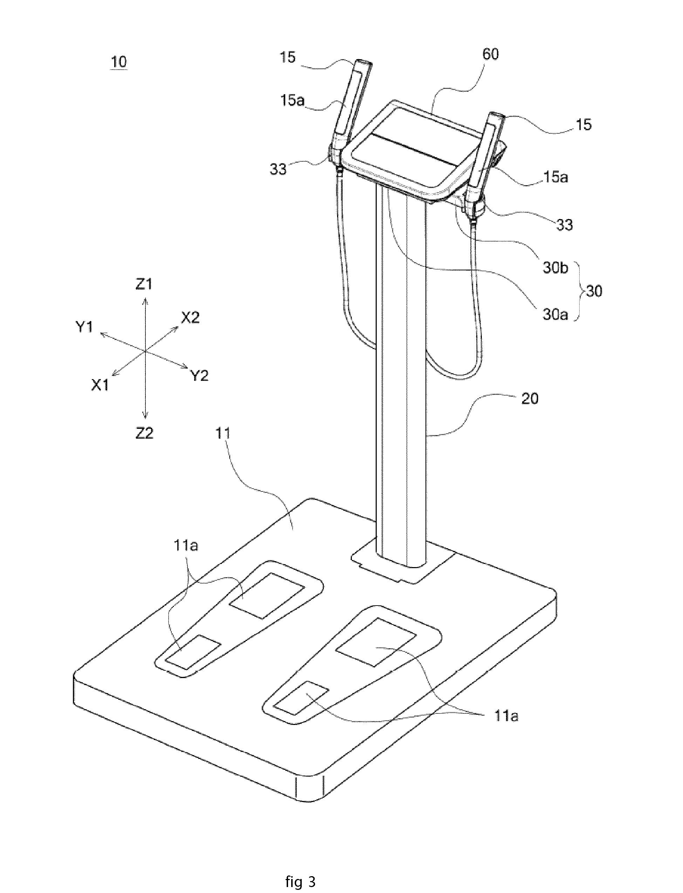

FIG. 3 shows an overall perspective view of the body composition monitor 10. As shown in FIG. 3, the body composition monitor 10 is provided with the measurement stand 11, the support column 20, the display unit holder 30, and the display unit 60. While details on the display unit holder 30 are mainly shown in FIG. 4, the display unit holder 30 is provided with a main holder unit 30a and hand-grip electrode storage units 30b. A roughly cylindrical hand-grip electrode unit 15 can be stored in a storage opening 33 in each hand-grip electrode storage unit 30b of the main holder unit 30a.

The measurement stand 11 achieves the functions of a typical body composition monitor and includes a platform provided on the upper surface thereof, a weight measurement circuit (load cell), an impedance measurement circuit, and the like. The result of measurement of body weight by the load cell is amplified, converted from analog to digital, and transmitted to a predetermined control device (microcomputer). Similarly, the result of impedance measurement by electrodes 11a formed on the upper surface (platform) of the measurement stand 11 and by electrodes 15a in the hand-grip electrode units 15 is converted from analog to digital and transmitted to the predetermined control device (microcomputer). The body weight, body fat percentage, physical age, and the like calculated by the control device (microcomputer) are transmitted to the display unit 60 and displayed on the display screen located on the display unit upper surface 61. Note that the present embodiment may also be adopted when the body composition monitor 10 is constituted by separate portions for the platform and for achieving the measurement functions of the measurement stand 11.

The support column 20 is fixed at a predetermined position in the center of the front of the measurement stand 11 (in the direction of the arrow X2) and rises vertically upward (in the direction of the arrow Z1).

The upper edge of the support column 20 and a holder bottom surface 32 of the display unit holder 30 are connected by, for example, a screw or a bolt, so as to secure the display unit holder 30 to the support column 20. The display unit 60 is connected to the display unit holder 30. As described above, the display unit 60 can be connected to face either forward or backward. Furthermore, the display unit 60 can slide back and forth (in the direction of the arrows X1 and X2), even when the direction of the display unit 60 is reversed.

FIG. 4 is an oblique perspective view of the display unit holder 30 from above. As described above, the display unit holder 30 is provided with the main holder unit 30a and two hand-grip electrode storage units 30b. These units are, for example, formed integrally from resin. When viewed from above, the main holder unit 30a has a roughly rectangular shape. The hand-grip electrode storage units 30b extend from the left and right of the main holder unit 30a in a roughly triangular shape. One of the storage openings 33 is formed at the tip of each of the hand-grip electrode storage units 30b as a cylindrical opening that can store one of the hand-grip electrode units 15. Here, the back side of each storage opening 33 (in the direction of the arrow X1) is cut away to allow for passage of a cable connected to the hand-grip electrode unit 15.

The holder connector 40 is formed on a holder upper surface 31 of the main holder unit 30a. The holder connector 40 and the below-described display unit connector 70 (see, for example, FIG. 5), provided on the display unit 60, are slidably connected.

A connector concavity 41 of a predetermined depth is formed in the main holder unit 30a and has a rectangular shape when viewed from above (from the arrow Z1, towards the arrow Z2). The back side of the connector concavity 41 (in the direction of the arrow X1) extends to near the back outer edge of the display unit holder 30. At the front of the connector concavity 41 (in the direction of the arrow X2), the wall is cut away. Note that except for predetermined structures to the left and right (front side-latch portions 44), portions of the bottom of the front of the connector concavity 41 (in the direction of the arrow X2) not necessary for connection are removed.

Side walls 42 on the left and right of the connector concavity 41 extend longitudinally in a straight line and function as guides for forward and backward movement of the display unit 60. While described in detail below, a distance D1 between the two side walls 42 is slightly wider than widths d1 and d2 of the display unit connector 70 (front latch hole placement convexity 71 and back latch hole placement convexities 76) in the display unit 60. As a result, the display unit connector 70 (the front latch hole placement convexity 71 and the back latch hole placement convexities 76) can slide back and forth smoothly with the side walls 42 as guides.

At the two back corners of the connector concavity 41, back side-latch portions 43 are formed inwards from the upper edge of the concavity, as shown in FIG. 4. In other words, the back side-latch portions 43 are shaped as claws. At the two front corners of the connector concavity 41, the front side-latch portions 44 are formed as claws like the back side-latch portions 43. The back side-latch portions 43 and the front side-latch portions 44 latch by fitting into back latch hole portions 77 or front latch hole portions 72, described below, in the display unit connector 70 of the display unit 60. The latching between these components is described below.

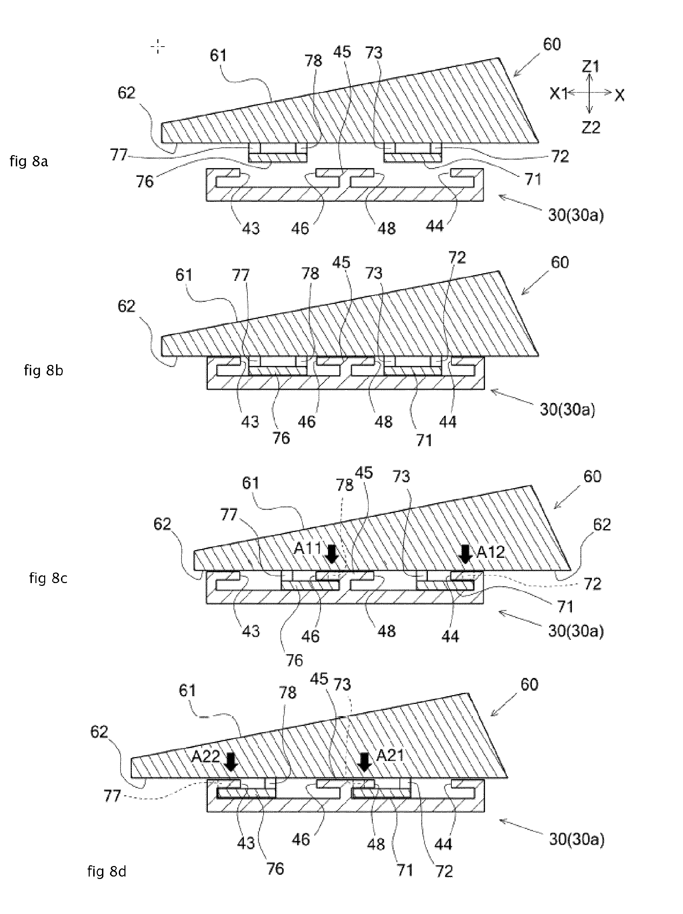

Furthermore, at approximately the center of the connector concavity 41 in the longitudinal direction, two bilaterally symmetric central latch portions 45 are formed. A cross-section of each central latch portion 45 is T-shaped. The upper edge portion of the cross-section is thus claw-shaped in the longitudinal direction. The front of each latch claw (in the direction of the arrow X2) becomes a central front latch portion 48, whereas the back of the latch claw (in the direction of the arrow X1) becomes a central back latch portion 46. An opening 47 is formed beneath each central back latch portion 46. Similarly, an opening 49 is formed beneath each central front latch portion 48. Note that the cross-sectional structure of the central latch portions 45 is also illustrated in the simplified schematic drawing of FIG. 8A, described below.

In the holder upper surface 31 of the main holder unit 30a, back latch holes 51 towards the back and front latch holes 52 towards the front are aligned longitudinally in outer regions to the left and to the right of the connector concavity 41. Positioning bias portions 69, described below, in the display unit 60 selectively fit into the back latch holes 51 and the front latch holes 52.

FIG. 5 is a perspective view of the display unit 60 from below. As shown in FIG. 5, the display unit 60 is provided with a grip bar 63 at the front for ease of use. At a display unit bottom surface 62 of the display unit 60, the positioning bias portions 69, the front latch hole placement convexity 71, and the back latch hole placement convexities 76 constitute the display unit connector 70. In terms of relative position, the front latch hole placement convexity 71 is towards the front (in the direction of the arrow X2), and the back latch hole placement convexities 76 are towards the back (in the direction of the arrow X1).

Specifically, the front latch hole placement convexity 71 is bilaterally symmetric and is shaped as a rectangular solid that is longer in the direction of width. As described above, the width d1 of the front latch hole placement convexity 71 is slightly narrower than the interval D1 between the side walls 42 of the holder connector 40. Furthermore, the height of the front latch hole placement convexity 71 is approximately equal to the depth of the connector concavity 41 of the display unit holder 30. Therefore, when the holder connector 40 and the display unit connector 70 are connected, the lower edge face of the front latch hole placement convexity 71 and the bottom surface of the connector concavity 41 are either in contact or are separated by a slight gap.

At the two front corners of the front latch hole placement convexity 71, the front latch hole portions 72 are formed as spaces where the front and side wall portions are cut away, as shown in FIG. 5. The back side-latch portions 43 and the front side-latch portions 44 selectively fit into the front latch hole portions 72. Towards the inside of the display unit 60 (in the direction of the arrow Z1), the front latch hole portions 72 constitute openings 74b.

Two back latch hole portions 73 are formed at predetermined positions on the back wall of the front latch hole placement convexity 71. The central latch portions 45 (central back latch portions 46 or central front latch portions 48) of the holder connector 40 fit into the back latch hole portions 73. Towards the inside of the display unit 60 (in the direction of the arrow Z1), the back latch hole portions 73 constitute openings 74a.

A pair of two back latch hole placement convexities 76 have the same shape as the front latch hole placement convexity 71 with the central portion thereof cut away. In other words, at the display unit bottom surface 62, two back latch hole placement convexities 76 are formed, one to the left and one to the right, back from the front latch hole placement convexity 71 (in the direction of the arrow X1) and to the same height as the front latch hole placement convexity 71. The back latch hole placement convexities 76 are bilaterally symmetric and have front latch hole portions 78 formed in the front walls. The distance d2 from the left side of the left back latch hole placement convexity 76 to the right side of the right back latch hole placement convexity 76 is the same as the width d1 of the front latch hole placement convexity 71 and is slightly narrower than the interval D1 between the side walls 42 of the holder connector 40.

The central latch portions 45 (central back latch portions 46 or central front latch portions 48) of the holder connector 40 fit into the front latch hole portions 78, in the same way as into the back latch hole portions 73. The upper portions of the front latch hole portions 78 (towards the inside of the display unit 60, in the direction of the arrow Z1) constitute openings 79b.

The back latch hole portions 77 are formed at the back outer corners of the back latch hole placement convexity 76. Specifically, one back latch hole portion 77 is formed at the back left corner of the left-hand back latch hole placement convexity 76. Similarly, the other back latch hole portion 77 is formed at the back right corner of the right-hand back latch hole placement convexity 76. The upper portions of the back latch hole portions 77 (towards the inside of the display unit 60, in the direction of the arrow Z1) constitute openings 79a. The back side-latch portions 43 and the front side-latch portions 44 of the holder connector 40 selectively fit into the back latch hole portions 77.

The positioning bias portions 69 can flex in the vertical direction and, when the display unit 60 slides back and forth, are pushed against by the holder upper surface 31 of the display unit holder 30 so as to be located within the display unit 60. When the display unit holder 30 is shifted backwards, the positioning bias portions 69 fit into the back latch holes 51. Conversely, when the display unit holder 30 is shifted forwards, the positioning bias portions 69 fit into the front latch holes 52. The shift positions of the display unit 60 are thus determined. Note that in the present embodiment, the display unit 60 shifts back and forth between two positions, but the number of positions between which the display unit 60 shifts back and forth may be increased by forming more latch holes like the back latch holes 51 and the front latch holes 52.

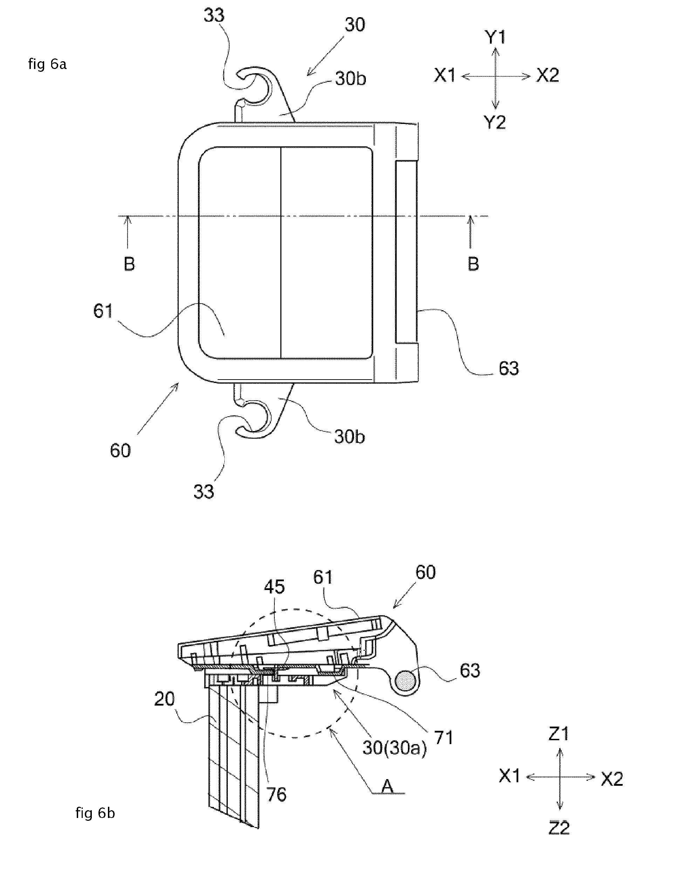

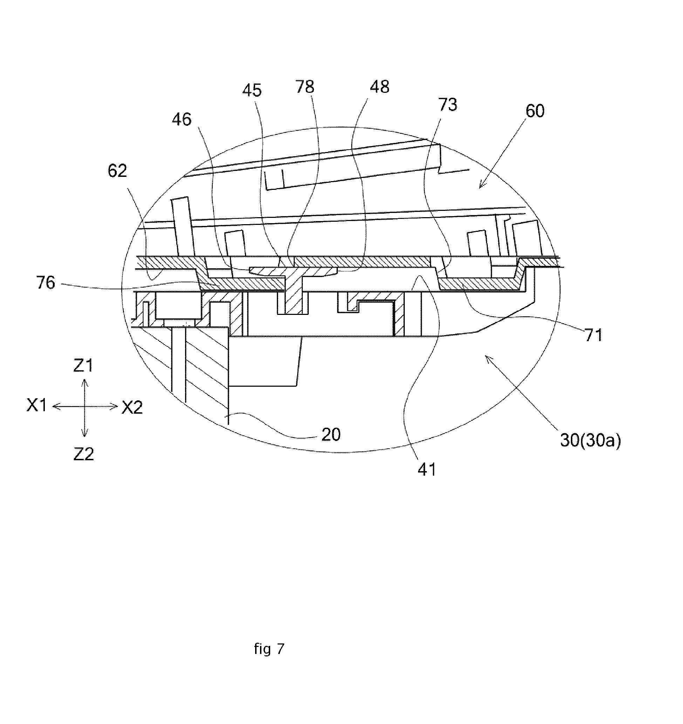

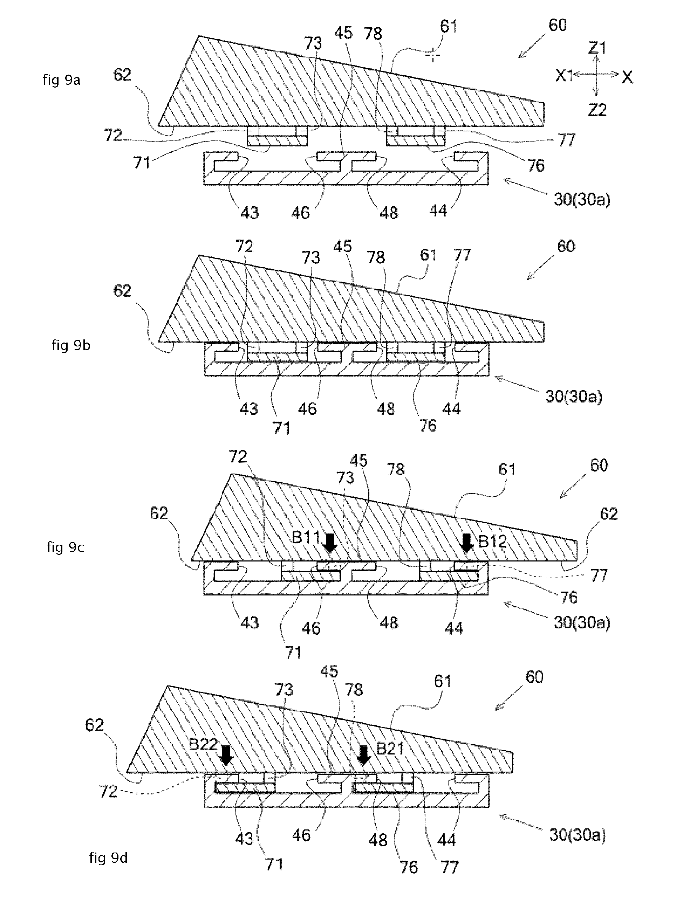

Next, mainly with reference to FIGS. 4-9D, the state of connection between the display unit holder 30 and the display unit 60 is described. FIG. 6A is a plan view of the state of connection of the display unit holder 30 and the display unit 60, and FIG. 6B is a B-B cross-sectional diagram of FIG. 6A. The state of connection in FIGS. 6A and 6B shows the display unit 60 facing forward and shifted towards the front. FIG. 7 shows an expanded view of region A in FIG. 6B. Furthermore, FIGS. 8A-8D schematically shows the structure when the display unit 60 is connected facing forward and is shifted back and forth, focusing on the structure for connection. FIGS. 9A-9D schematically shows the structure when the display unit 60 is connected facing backward and is shifted back and forth, focusing on the structure for connection. Note that for the sake of convenience, in FIGS. 8A-8D and 9A-9D the structure for connecting the display unit holder 30 and the display unit 60 is shown arranged linearly.

First, the steps for connecting the display unit 60 to face forward are described. As shown in FIG. 8A, the display unit 60 is positioned above the display unit holder 30. Next, as shown in FIG. 8B, the display unit 60 is placed so that the central latch portions 45 are located between the front latch hole placement convexity 71 and the back latch hole placement convexities 76. The display unit 60 is then shifted in the desired direction to determine the position towards the front or the back.

Here, as shown in FIG. 8C, when the display unit 60 is positioned towards the front (in the direction of the arrow X2), the central back latch portions 46 of the central latch portions 45 fit into the front latch hole portions 78 of the back latch hole placement convexities 76 (arrow A11). The front side-latch portions 44 fit into the front latch hole portions 72 of the front latch hole placement convexity 71 (arrow A12).

As shown in FIG. 8D, when the display unit 60 is positioned towards the back (in the direction of the arrow X1), the central front latch portions 48 of the central latch portions 45 fit into the back latch hole portions 73 of the front latch hole placement convexity 71 (arrow A21). The back side-latch portions 43 fit into the back latch hole portions 77 of the back latch hole placement convexities 76 (arrow A22).

Next, the steps for connecting the display unit 60 to face backward are described. When the display unit 60 is connected facing forward, the state of connection may be undone by following the steps in FIGS. 8A-8D above in reverse. As shown in FIG. 9A, the display unit 60 is then positioned above the display unit holder 30 facing backward. Next, as shown in FIG. 9B, the display unit 60 is placed so that the central latch portions 45 are located between the front latch hole placement convexity 71 and the back latch hole placement convexities 76. At this point, contrary to the state shown in FIG. 8B, the front latch hole placement convexity 71 is positioned towards the back (in the direction of the arrow X1), and the back latch hole placement convexities 76 are towards the front (in the direction of the arrow X2). The display unit 60 is then shifted in the desired direction to determine the position towards the front or the back.

Here, as shown in FIG. 9C, when the display unit 60 is positioned towards the front (in the direction of the arrow X2), the central back latch portions 46 of the central latch portions 45 fit into the back latch hole portions 73 of the front latch hole placement convexity 71 (arrow B11). The front side-latch portions 44 fit into the back latch hole portions 77 of the back latch hole placement convexities 76 (arrow B12).

As shown in FIG. 9D, when the display unit 60 is positioned towards the back (in the direction of the arrow X1), the central front latch portions 48 of the central latch portions 45 fit into the front latch hole portions 78 of the back latch hole placement convexities 76 (arrow B21). The back side-latch portions 43 fit into the front latch hole portions 72 of the front latch hole placement convexity 71 (arrow B22).

In this way, the connection between the display unit holder 30 and the display unit 60 allows for the display unit 60 to slide back and forth even when facing backwards, while appropriately maintaining the state of connection. Accordingly, even in cases such as when the user is well built, measurement can be performed from an appropriate position. Furthermore, visibility of the display unit 60 can be maintained even when the viewer is someone other than the person being measured. For example, the display unit 60 can be used as follows. In the state shown in FIG. 2B, a person standing in front (in the direction of the arrow X2) of the body composition monitor 10 who is explaining the measurement results to the person being measured can detach the display unit 60, with display of the measurement results being maintained, and replace it as shown in FIG. 2A.

The present invention has been described based on the above embodiment. The embodiment is simply an example, however, and those skilled in the art will understand that combinations of the above structural elements and various modifications thereof are possible, and that such modifications are within the scope of the present invention.

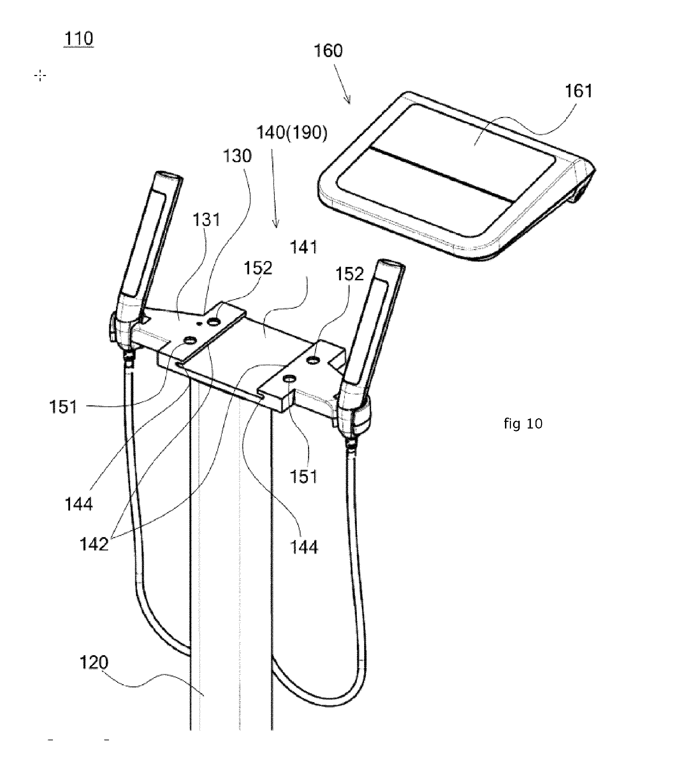

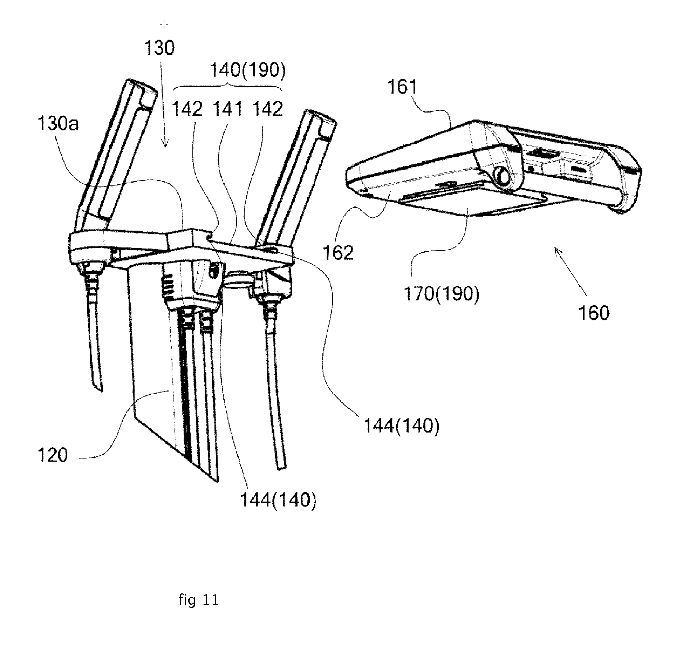

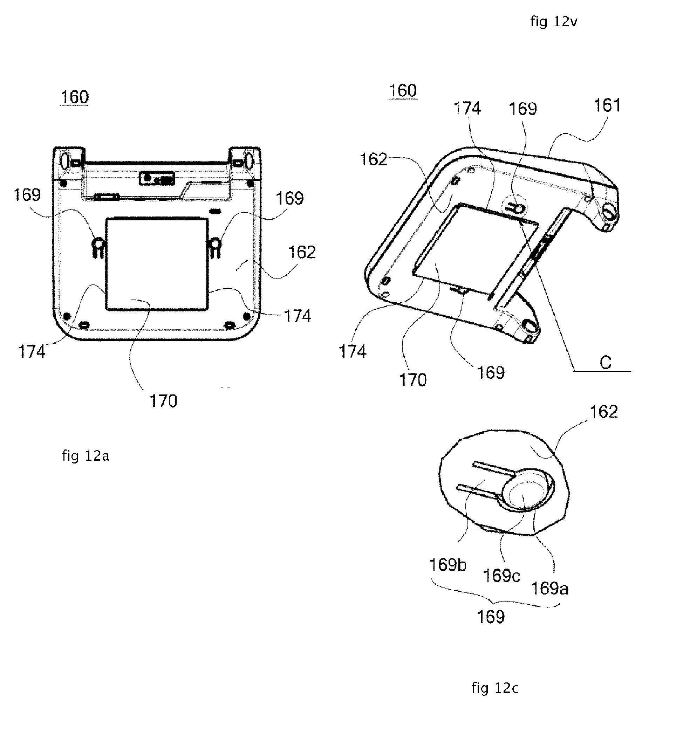

FIGS. 10-12C show a body composition monitor 110 according to a modification. FIG. 10 is a perspective view of the body composition monitor 110, showing the display unit 60 separated from the display unit holder 30. Specifically, FIG. 10 shows a holder connector 140 and a display unit connector 170, which form a sliding connector 190, separated from each other. Note that a support column 120, a display unit holder 130, and a display unit 160 are also shown, whereas the measurement stand is omitted from the figures. FIG. 11 is a front perspective view of the display unit 160 and the display unit holder 130 from below. FIG. 12A shows the bottom surface of the display unit 160, FIG. 12B is a back perspective view from below, and FIG. 12C is an enlarged view of region C (positioning bias portion 169) in FIG. 12B.

Specifically, a display unit connector 170 with a cross-sectional T shape is formed on a display unit bottom surface 162 of the display unit 160. The bottom surface of the display unit connector 170 is rectangular, and a cross-section thereof has the shape of an inverted T. Fit pieces 174 extend to the left and right to form the cross-sectional inverted T shape.

An elastic positioning bias portion 169 is formed on the display unit bottom surface 162 on each side of the display unit connector 170. As shown in FIG. 12C, each positioning bias portion 169 is formed by a slit-shaped opening 169a provided around a predetermined portion of the display unit bottom surface 162, an elongated base portion 169b, and a convex abutting portion 169c provided at the tip of the base portion 169b.

On the other hand, the holder connector 140 is formed on a holder upper surface 131 of the display unit holder 130. The holder connector 140 is formed by a connector concavity 141, back latch holes 151, and front latch holes 152. As shown in the figures, the connector concavity 141 is formed to pass completely through the holder upper surface 131 by removal of the front and back walls of the holder upper surface 131. Furthermore, fitting grooves 144 are respectively formed in left and right side walls 142 of the connector concavity 141. The fit pieces 174 fit into the fitting grooves 144 so as to be able to slide back and forth. The fitting grooves 144 and the fit pieces 174 thus fulfill a guide function during movement back and forth.

On the holder upper surface 131, the back latch holes 151 and the front latch holes 152 are aligned longitudinally in regions outside the connector concavity 141 to the left and right thereof. The abutting portions 169c of the positioning bias portions 169 latch into the back latch holes 151 and the front latch holes 152. In other words, the positioning bias portions 169, the back latch holes 151, and the front latch holes 152 are used as a positioning means during sliding back and forth.

With this structure as well, the display unit 160 can be connected to the display unit holder 130 while facing either forward or backward. The display unit connector 170 can thus be fit into the holder connector 140, allowing for connection and movement, regardless of whether the display unit 160 faces forward or backward.

In the above embodiment and modification, the display units 60 and 160 are respectively connected to the display unit holders 30 and 130. The present invention is not limited to this configuration, however, and a structure for achieving the functions of the display unit holders 30 and 130 may be provided directly on the support columns 20 and 120. Furthermore, the display units 60 and 160 are not limited to being attached to the support columns 20 and 120, and may instead be attached to the measurement stand 11. In other words, a display unit holder portion with the same structure as the holder connector 40 in the display unit holder 30 or the like may be formed on the measurement stand 11. The support column 20 is also not limited to being directly attached to the measurement stand 11, and instead may be arranged alongside the measurement stand 11 with a predetermined connector member therebetween.

**REFERENCE SIGNS LIST**

| | |
|---|---|
| 10, 110: | Body composition monitor |
| 11: | Measurement stand |
| 11a, 15a: | Electrode |
| 15: | Hand-grip electrode unit |
| 20, 120: | Support column |
| 30, 130: | Display unit holder |
| 30a: | Main holder unit |
| 30b: | Hand-grip electrode storage unit |
| 31, 131: | Holder upper surface |
| 33: | Storage opening |
| 40, 140: | Holder connector (second connecting element) |
| 41, 141: | Connector concavity |
| 42, 142: | Side wall |
| 43: | Back side-latch portion |
| 44: | Front side-latch portion |
| 45: | Central latch portion |
| 46: | Central back latch portion |
| 47, 49, 74a, 74b, 79a, 79b: | Opening |
| 48: | Central front latch portion |
| 51, 151: | Back latch hole |
| 52, 152: | Front latch hole |
| 60, 160: | Display unit |
| 61, 161: | Display unit upper surface |
| 62, 162: | Display unit bottom surface |
| 63: | Grip bar |
| 69, 169: | Positioning bias portion |
| 70, 170: | Display unit connector (first connecting element) |
| 71: | Front latch hole placement convexity |
| 72: | Front latch hole portion |
| 73: | Back latch hole portion |
| 76: | Back latch hole placement convexity |
| 77: | Back latch hole portion |
| 78: | Front latch hole portion |
| 90, 190: | Sliding connector |
| 144: | Fitting groove |
| 174: | Fit piece |

## Claims

1. A biological information measuring device comprising:
a platform on which a user stands for measurement;
a display unit disposed at an upper side of the platform; and
a sliding connector connecting the display unit to the upper side of the platform so that the display unit slides back and forth to predetermined positions.

2. The biological information measuring device of claim 1, wherein the sliding connector allows for reversal of a state of connection of the display unit, the display unit sliding back and forth in the reversed state.

3. The biological information measuring device of claim 1, wherein the sliding connector includes a guide portion that guides sliding of the display unit back and forth.

4. The biological information measuring device of claim 3, further comprising:
a support column provided on the platform, and a display unit holder provided on the support column and receiving the display unit, wherein
the sliding connector includes a first connecting element provided on the display unit and a second connecting element provided on the display unit holder, the guide portion is provided on the display unit holder, and the first connecting element and the second connecting element latch by a latch claw, provided on one of the first connecting element and the second connecting element, fitting into an opening provided on the other of the first connecting element and the second connecting element and configured to engage with the latch claw.

5. The biological information measuring device of claim 1, further comprising:
a display unit holder holding the display unit at the upper side of the platform, wherein
the sliding connector includes a front latch hole placement convexity, a back latch hole placement convexity, a central latch portion, a front side-latch portion, and a back side-latch portion, the front latch hole placement convexity and the back latch hole placement convexity being provided on the display unit, and the central latch portion, the front side-latch portion and the back side-latch portion being provided on the display unit holder,
when the display unit is held in the display unit holder facing forward and the display unit is slid towards a front of the platform, a back latch portion of the central latch portion fits into a front latch hole portion of the back latch hole placement convexity, and the front side-latch portion fits into a front latch hole portion of the front latch hole placement convexity, and
when the display unit is held in the display unit holder facing forward and the display unit is slid towards a back of the platform, a front latch portion of the central latch portion fits into a back latch hole portion of the front latch hole placement convexity, and the back side-latch portion fits into a back latch hole portion of the back latch hole placement convexity.

6. The biological information measuring device of claim 5, wherein when the display unit is held in the display unit holder facing backward and the display unit is slid towards the front of the platform, the back latch portion of the central latch portion fits into the back latch hole portion of the front latch hole placement convexity, and the front side-latch portion fits into the back latch hole portion of the back latch hole placement convexity, and
when the display unit is held in the display unit holder facing backward and the display unit is slid towards the back of the platform, the front latch portion of the central latch portion fits into the front latch hole portion of the back latch hole placement convexity, and the back side-latch portion fits into the front latch hole portion of the front latch hole placement convexity.
